# EUROPEAN PATENT SPECIFICATION

(11) **EP 3 744 355 B1**
(45) Date of publication and mention of the grant of the patent: **21.12.2022**
(21) Application number: 19382434.9
(22) Date of filing: 29.05.2019
(51) Int. Cl.: A61L 27/30, A61L 27/54

(54) **COATING FOR NON-PERMANENT TRANSCUTANEOUS IMPLANT**
BESCHICHTUNG FÜR NICHTPERMANENTES TRANSKUTANES IMPLANTAT
REVÊTEMENT POUR IMPLANT TRANSCUTANÉ NON-PERMANENT

(43) Date of publication of application: 02.12.2020
(73) Proprietor: Metal Estalki, S. L., 48170 Zamudio, Bizkaia (ES)
(72) Inventor: Azkona Villaverde, Ibon, 48170 Zamudio, Bizkaia (ES); Fernández de Larrinoa Estefanía, Javier, 48170 Zamudio, Bizkaia (ES); Braceras Izaguirre, Iñigo, 48160 Derio, Bizkaia (ES); Alvarez Luque, Noelia, 48160 Derio, Bizkaia (ES)
(74) Representative: Balder IP Law, S.L.

(56) References cited:
- US-A- 5 697 779
- US-A1- 2016 106 541
- ZHAO ET AL: "Bactericidal and corrosive properties of silver implanted TiN thin films coated on AISI317 stainless steel", SURFACE AND COATINGS TECHNOLOGY, ELSEVIER BV, AMSTERDAM, NL, vol. 201, no. 9-11, 31 January 2007 (2007-01-31), pages 5676-5679, XP005738744, ISSN: 0257-8972, DOI: 10.1016/J.SURFCOAT.2006.07.172
- KELLY P J ET AL: "A study of the antimicrobial and tribological properties of TiN/Ag nanocomposite coatings", SURFACE AND COATINGS TECHNOLOGY, ELSEVIER BV, AMSTERDAM, NL, vol. 204, no. 6-7, 25 December 2009 (2009-12-25), pages 1137-1140, XP026770405, ISSN: 0257-8972, DOI: 10.1016/J.SURFCOAT.2009.05.012 [retrieved on 2009-05-14]

## Description

### FIELD OF THE INVENTION

The present invention relates to the field of medical implants, in particular, to a coating for a non-permanent transcutaneous implant comprising TiₓAg_{y}N_{z}. The process for its manufacturing and its medical application, e.g. in bone fractures, are also disclosed.

### BACKGROUND OF THE INVENTION

Implants are medical devices that are placed inside or on the surface of a body, typically made from metal, plastic, ceramic among other suitable materials. Many implants are prosthetics, intended to replace missing body parts. Other implants provide support to organs and tissues.

On the one hand, implants can be placed permanently or they can be removed once they are no longer needed. For example, stents or hip implants are intended to be permanent, but chemotherapy ports or screws to repair broken bones can be removed when they are no longer needed, and therefore considered or referred to as "non-permanent" implants.

In temporary therapies where the implant must be removed at the end of the treatment without causing side effects (damages) in the bone tissue, titanium is not suitable as material for implants. In fact, titanium implants, in comparison with stainless steel alloy implants, stick intimately to the bone, i.e. they have a greater osseointegration, and it is difficult to remove them from a bone tissue.

On the other hand, the prevalence of infections is still a big concern in many implants. For this reason, antibacterial and biocompatible coatings for implants comprising silver, intended to be in direct contact with a human or animal body, have been developed. Some of them, make use of the Physical Vapour Deposition (PVD) technology for the deposition of the coatings.

For example, US2013224274A describes a medical product (medical tools and instruments) coated with a sequence of layers of TiN alternated with bactericidal Ag/TiN layers, and wherein the thickness of a layer and the concentration of Ag can vary along the sequence of layers. The presence of this sequence of layers increases the adhesiveness of the coating to the medical product.

CN107190234A describes a bactericidal TiN/Ag nano-multilayer film deposited in an Ti₆Al₄V alloy by multi-arc ion plating (multi-AlP) and composed by different layers of TiN alternated with Ag layers. This film can be deposited on artificial joints or dental implants.

EP2240212B describes a biomedical article (orthopaedic implants, e.g. prosthetic hips, knees, shoulders, and tools and instruments) having a first silver-containing metal nitride coating and a second silver-containing metal nitride coating on the outer surface of the first coating. The second coating has a greater amount of silver than the first coating. Such an arrangement provides a strong anti-microbial effect at the surface. Moseke et al. describe in Trans. Electr. Electron. Mater., 15 (5) 275 (2014), "Hard implant coatings with antimicrobial properties" a bactericidal coating for implants made by TiN/Ag by the combination of arc evaporation and magnetron sputtering method. The combined use of said methods leads to the formation of a layer with two different silver phases that allows a prolonged silver ion release in the body.

Kang et al. describe in J. Mater. Sci.: Mater Med. (2011), 22:2711-2720 "Antibacterial Properties of TiAgN and ZrAgN thin film coated by physical vapour deposition for medical applications" a bactericidal coating with a layer of TiAgN deposited on a pure titanium substrate by arc ion plating (AIP) method.

However, the previous above mentioned developments have not considered that the implants must eventually be removed in some applications (non-permanent implants) and should have a degree of integration into bone tissue (degree of osseointegration) as low as possible.

In view of the above, there is the need in the state of the art of providing a biocompatible, and antibacterial transcutaneous implant with substantially the same low-degree of osseointegration as a stainless steel implant for use in temporal therapies, e.g. as non-permanent implant, in a human or animal body. US5697779 A discloses a temporary implant positioned in a nonocclusal surface of the maxillary or mandibular jawbone. The implant may be made of a metal, a ceramic, a bioresorbable material. Preferred metals include titanium, titanium based alloys, etc. There is little or no bone integration required for the implant. Because the implant is designed to have little bone integration, it is significantly easier to remove after treatment is completed. In most cases, where little to no bone integration is desired, it is preferred to have a smooth bone-contacting outer body surface. The outer body surface may be chemically or mechanically treated to increase or decrease surface roughness and bone integration. The temporary implant may be coated with a material to inhibit or prevent infection, such as an antibiotic.

### DESCRIPTION OF THE DRAWINGS

The present invention will be further described by way of example with reference to the drawings, in which:

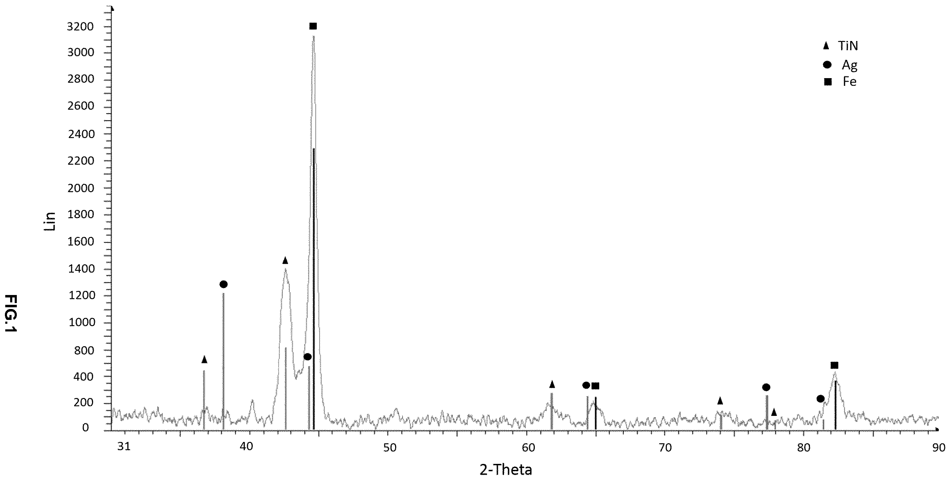

Figure 1 is a X-ray diffraction (XRD) pattern calculated in a 2-Theta range comprised between 30 and 90 of a coating of the invention described in the example 1 (coated implant L10).

### DESCRIPTION OF THE INVENTION

The present coating for a non-permanent transcutaneous implant, hereinafter referred to as coating of the invention, confers to the implant a bactericide property maintaining a low degree of osseointegration that makes it suitable for use in a human or animal body as temporary (non-permanent) implant. Moreover, the composition of the coating of the invention makes it biocompatible.

In a first aspect, the present disclosure relates to a coating for a non-permanent transcutaneous implant comprising TiₓAg_{y}N_{z} characterized in that:
a) (y/ (x+y)) is comprised between 0.0025 and 0.20;
b) the sum of x+y+z is equal to 1;
c) z is comprised between 0 and 0.55;
   wherein x, y and z denote respectively the atomic ratios of Ti, Ag and N; and wherein the coating has a thickness comprised between 0.1 µm and 0.45 µm; and
   wherein the coating has a roughness of Ra comprised between 0.01 and 0.35 µm.

The atomic ratios of Ti, Ag, N have been determined by energy-dispersive X-ray spectroscopy (EDS) analysis and X-Ray photoelectron spectrometry (XPS), methods of analysis known by the skilled person. The sum of the atomic ratios x, y and z is 1, excluding unavoidable impurities, that corresponds to a total atomic percentage of 100 % at. of the TiₓAg_{y}N_{z}.

In a particular embodiment, the atomic ratio of the unavoidable impurities is at most 0.005, preferably at most 0.0025, still more preferably at most 0.0005 (which corresponds to an atomic percentage of preferably at most 0.5% at., more preferably at most 0.25% at., still more preferably 0.05% at. of to the total atomic percentage of the TiₓAg_{y}N_{z} respectively).

Typical unavoidable impurities are, for example, silver oxide or titanium oxide.

In a particular embodiment, z is comprised between 0.03 and 0.55, or between 0.2 and 0.55, or between 0.25 and 0.45, or between 0.3 and 0.4. In another particular embodiment, z is comprised between 0.05 and 0.1.

In a yet particular embodiment, (y/(x+y)) is comprised between 0.0025 and 0.15, or between 0.0025 and 0.10, or between 0.005 and 0.050, or between 0.0075 and 0.025. In a further particular embodiment, the coating of the invention has a thickness comprised between 0.15 and 0.45 µm, or between 0.2 and 0.40 µm or between 0.25 and 0.35 µm or 0.3 µm.

In an additional particular embodiment, the coating of the invention has a roughness of Ra comprised between 0.01 and 0.30 µm, or between 0.01 and 0.28 µm or between 0.01 and 0.25 µm. In another particular embodiment, the coating of the invention has a roughness of Ra comprised between 0.025 and 0.30 µm or between 0.035 and 0.28 µm or between 0.04 and 0.25 µm.

Any of the above defined values of z and y/(x+y) in combination with any of the above defined values of thickness and any of the roughness values are to be understood as defined herein.

Thus, in some exemplary embodiments, the coating of the invention comprises TiₓAg_{y}N_{z} characterized in that:
a) (y/ (x+y)) is comprised between 0.02 and 0.045;
b) the sum of x+y+z is equal to 1;
c) z is comprised between 0 and 0.55;
   wherein x, y and z denote respectively the atomic ratios of Ti, Ag and N; and wherein the coating has a thickness comprised between 0.25 µm and 0.35 µm; and wherein the coating has a roughness of Ra comprised between 0.04 and 0.25 µm.

In another exemplary embodiment, the coating of the invention comprises TiₓAg_{y}N_{z} characterized in that:
a) (y/ (x+y)) is comprised between 0.016 and 0.077;
b) the sum of x+y+z is equal to 1;
c) z is comprised between 0 and 0.55;
   wherein x, y and z denote respectively the atomic ratios of Ti, Ag and N; and wherein the coating has a thickness comprised between 0.1 µm and 0.2 µm; and wherein the coating has a roughness of Ra comprised between 0.037 and 0.094 µm.

In a second aspect, the present disclosure relates to a process for producing a coating for a non-permanent transcutaneous implant comprising the steps of:
a) evaporating an Ag alloyed Ti target in the presence of a gas selected from nitrogen, an inert gas, and mixtures thereof, obtaining TiₓAg_{y}N_{z};
b) coating an implant until a thickness comprised between 0.1 µm and 0.45 µm with the TiₓAg_{y}N_{z} obtained in step a) by arc ion plating technology to obtain a coated implant;
c) removing surface metal droplets formed in step b) from the coated implant until a roughness of Ra comprised between 0.01 and 0.35 µm is achieved.

Step a) is performed evaporating an Ag alloyed Ti target under a gas atmosphere wherein said gas is selected from nitrogen, an inert gas, and mixtures thereof.

The Ag alloyed Ti target defined in the present disclosure may be manufactured according to conventional methods, i.e. casting or powder metallurgy.

In the context of the present invention, the expression "inert gas" refers to a gas which does not undergo any chemical reactions under the given conditions of the process of the invention. In a preferred embodiment, the inert gas is Argon.

In a particular embodiment, the evaporation of an Ag alloyed Ti target is carried out under vacuum at a working pressure of 0.1 to 5 Pa of nitrogen during a period of 0.1 and 240 minutes and at a temperature comprised between 150 and 500 °C, or during a period of 0.5 and 10 minutes and at temperature comprised between 350 and 420 °C. In another particular embodiment, the evaporation of an Ag alloyed Ti target is carried out under vacuum at a working pressure of 0.1 to 5 Pa of Argon during a period of 10 and 60 minutes and at a temperature comprised between 150 and 500°C, or during a period of 15 and 25 minutes and at temperature comprised between 350 and 420 °C.

In principle step b) can be carried out on any transcutaneous implant, without limitations, made in any acceptable material conventionally used in implants.

Prior to the coating of step b), the transcutaneous implant is ultrasonically cleaned in a solvent such as acetone, alcohol, a suitable detergent and distilled water and then dried.

To perform the arc ion plating method of step b), the transcutaneous implant is placed on a rotational substrate holder for the deposition, at a certain distance from the TiₓAg_{y}N_{z}.

In a particular embodiment, the distance between the transcutaneous implant and the TiₓAg_{y}N_{z} is comprised between 80 and 500 mm or 100 and 200 mm.

The arc ion plating method of step b) is carried out at a pressure comprised between 0.1 and 5 Pa, or 0.3 and 4 Pa, or 0.3 and 3 Pa or 0.5 and 2 Pa.

In another particular embodiment, the arc ion plating method of step b) is performed with a deposition time comprised between 0.3 and 20 minutes.

The arc ion plating method of step b) is carried out with an arc current comprised between 40 and 190 A and a bias voltage comprised between 20 and 200 V.

In a particular embodiment, the arc power of the arc ion plating method of step b) is comprised between 80 and 120 A and a bias voltage comprised between 60 and 120 V.

Step b) provides on a transcutaneous implant a coating with a thickness comprised between 0.1 and 0.45

µm as defined above, or between 0.15 and 0.45 µm, or between 0.2 and 0.40 µm or between 0.25 and 0.35 µm or 0.3 µm.

In a particular embodiment, the coating obtained after step b) is a single layer of TiₓAg_{y}N_{z}.

Step c) consists in removing surface metal droplets formed in step b). Exemplary methods used to remove metallic droplets are for example abrasive drag grinding, abrasive filament brushing, magnetic particle abrasion finishing, abrasive dry, wet blasting and mixtures thereof.

This step c) does not affect the antibacterial properties when the implant is used in the body. In a particular embodiment, the method used to remove metal droplets is an abrasive drag grinding method performed during a period of 6 minutes. In another particular embodiment, the method used to remove metal droplets is an abrasive drag grinding method performed during a period of 13 minutes.

After step c) the roughness value of Ra is comprised between 0.01 and 0.35 µm as defined above, or between 0.01 and 0.30 µm, or between 0.01 and 0.28 µm or between 0.01 and 0.25 µm.

In an exemplary embodiment, the process of the invention comprises the steps of:
a) evaporating an Ag alloyed Ti target in the presence of a gas selected from nitrogen, an inert gas, and mixtures thereof, obtaining TiₓAg_{y}N_{z};
b) coating an implant until a thickness comprised between 0.25 µm and 0.35 µm with the TiₓAg_{y}N_{z} obtained in step a) by arc ion plating technology to obtain a coated implant;
c) removing surface metal droplets formed in step b) from the coated implant until a roughness of Ra comprised between 0.04 and 0.25 µm is achieved.

As shown in Figure 1, results of XRD analysis of the coating of the exemplary embodiment has confirmed the presence of Ti mainly as nitride which grows as face centred cubic structure with a lattice parameter of 4.24 Angstrom.

In a third aspect, the present disclosure relates to a coated non-permanent transcutaneous implant comprising a transcutaneous implant and a coating as defined previously or obtained by the process for its manufacturing as previously defined.

The present TiₓAg_{y}N_{z} coating with the defined thickness and roughness (shown in experiments 1 and 2) has been shown to confer the low-degree of osseointegration to the implant and different samples. Due to the low-degree of osseointegration, the coated implant can be easily removed from the human or animal body site once therapy is completed. For example, the coated implant of the invention is used in the treatment of a bone fracture and it is removed once the fracture has healed without causing damages to the bone tissue.

As already mentioned, the low-degree of osseointegration is substantially the same as the degree of osseointegration of a stainless steel alloy, in particular the stainless steel AISI 316L as shown in the example 1.

In a particular embodiment, the transcutaneous implant material can be any conventional biomaterial such as stainless steel alloys, commercially pure Ti, Titanium grade 5, titanium alloys, biocompatible CoCr alloys or Polyether ether ketones (PEEK) and mixtures thereof. In a particular embodiment, the transcutaneous implant material is stainless steel alloy, for example austenitic or a martensitic alloy.

The transcutaneous implant can have in principle any geometry and size without limitations. In a particular embodiment, the geometry of the transcutaneous implant can be flat, cylindrical or semi-spherical.

In another particular embodiment, the transcutaneous implant is cylindrical.

In another particular embodiment, the transcutaneous implant is cylindrical, 6 mm in diameter, 10 mm long and threaded at one end. In another particular embodiment, the size of a cylindrical transcutaneous implant is 2 mm of diameter and 45 mm long and threaded at one end.

The coated non-permanent transcutaneous implant can be coated by the coating of the invention on at least part, or, on all of its surface.

In an exemplary embodiment, the coated non-permanent transcutaneous implant comprises a TiₓAg_{y}N_{z} coating with a thickness comprised between 0.25 and 0.35 µm and a roughness of Ra comprised between 0.04 and 0.25 µm, and wherein y/(x+y) is comprised 0.02 and 0.045 and wherein the coating is on all the surface of the implant and wherein the implant has a cylindrical geometry and is threaded at one end.

Due to the low-degree of osseointegration of the coating of the invention, the coated transcutaneous implant can be used as non-permanent implant. Moreover, due to the presence of Ti and Ag within the coating, the coated transcutaneous implant shows biocompatibility and antibacterial properties.

Thus, in a further aspect, the present disclosure relates to a coated non-permanent transcutaneous implant for use in therapy.

In a particular embodiment, the coated non-permanent transcutaneous implant is for use in the treatment of bone fractures. The implant presents the advantage that after the end of the therapy, due to its low-degree of osseointegration, it can be removed without causing sides effects in the body site where the implant has been inserted. Moreover, the coated non-permanent transcutaneous implant is also for use in the prevention or treatment of osteomyelitis or bacterial infection.

In a further particular embodiment, bacterial infection is produced by bacteria that are selected from the group consisting of *Staphylococcus epidermis, Escherichia coli and Methicillin-resistant Staphylococcus aureus* (MRSA).

As it is demonstrated in the Examples explained here below, the coating applied on a transcutaneous implant confers it a low-degree of osseointegration and bactericidal properties.

At the same time, the coating is biocompatible and does not produce side effects in the human body.

The following examples are merely illustrative and should not be considered as limiting the invention.

### EXAMPLES

### Example 1

The present example discloses the preparation of the coating and its application to transcutaneous implants and samples in order to obtain coated non-permanent transcutaneous implants (hereinafter referred as coated implant L10) and coated samples. The medical use of the coated implant L10 was tested in vivo and the coated samples in vitro.

### 1.1. Preparation of TiₓAg_{y}N_{z}

An Ag alloyed Ti target with a ratio of 20/80 at.% was manufactured according to conventional methods, i.e. casting or powder metallurgy.

Then, the Ag alloyed Ti target was evaporated under a vacuum in the presence of N₂ gas during a period of 4 minutes and in a temperature of 380°C in order to provide TiₓAg_{y}N_{z}.

### 1.2. Coating a cylindrical transcutaneous implant and samples with TiₓAg_{y}N_{z}

Pins made of AISI 316L stainless steel with a cylindrically shaped geometry, i.e. 2 mm diameter, 45 mm long in size were used as transcutaneous implants. Flat samples of stainless steel with 50 mm x 50 mm x 1 mm in size were also used. Prior to the coating with TiₓAg_{y}N_{z} obtained in point 1.1., the cylindrical transcutaneous implants and samples were ultrasonically cleaned in isopropyl alcohol and finally dried.

Then, the transcutaneous implants and samples were coated with the TiₓAg_{y}N_{z} by arc ion plating method. The transcutaneous implant and samples were placed on a rotational substrate holder for the deposition. The distance between the transcutaneous implant or samples and the TiₓAg_{y}N_{z} was 100 mm. The process was carried out at a pressure of 0.8 Pa of nitrogen. The deposition time was 4 minutes. The arc current applied was 90 A and the bias voltage was 80 V.

The deposition of the TiₓAg_{y}N_{z} by arc ion plating method on the transcutaneous implant and samples provided a coating with a thickness comprised between 0.3 and 0.35 µm measured according with the standard ISO 26423:2016-11. Then, the coated transcutaneous implant and samples were subjected to removal, at the surface, of metal droplets formed during the arc PVD coating method. The method used to remove metal droplets was an abrasive drag grinding method, performed during 6 minutes. After this step, a coating roughness of Ra 0.196±0.032 µm was obtained for the coated implant L10 and 0.037±0.006 µm for the coated stainless steel polished flat samples. Surface roughness was measured by contact profilometry (Veeco Dektak 150).

The TiₓAg_{y}N_{z} coating had an average Ag content, measured by EDS comprised between 0,3 and 0,8 At.%. These data corresponded to a value of y/(x+y) [Ag / (Ag + Ti)] of 2.0 to 4.5 At.%., i.e., the atomic ratio was y/ (y+x) = 0.02 to 0.045.

XRD (X-Ray Diffraction) analysis spectrum of the coating showed the presence of silver mainly as a metallic compound, and titanium mainly as a nitride compound (Figure 1).

### 1.3. In vitro and in vivo antibacterial results and in vivo determination of the degree of osseointegration of the coated implant L10

The coated implants L10 were tested *in vivo* and coated stainless steel samples *in vitro* in order to prove their antibacterial properties (*in vitro* and *in vivo*) and in order to determine the degree of osseointegration of the implants in a femur of a rabbit (*in vivo*). The properties of the coated implants L10 and coated samples were compared with the properties of an uncoated transcutaneous implant (hereinafter referred to as the "uncoated implant") and uncoated samples used as control for comparative purposes. The uncoated implants were the same uncoated pins made of AISI 316L stainless steel with a cylindrically shaped geometry, i.e. 2 mm diameter, 45 mm long in size.

The uncoated samples were also made by stainless steel and with a flat geometry and with 50 mm x 50 mm x 1 mm in size.

### 1.3.1. In vitro assay: antibacterial property of the coated samples

The antibacterial activity of the coated samples was assessed *in vitro,* following the ISO 22196: 2011 (Measurement of antibacterial activity on plastics and other non-porous surfaces) against *Staphylococcus epidermidis.*

JIS Z 2801: 2000 standard (Antimicrobial products -Test for antimicrobial activity and efficacy) states that if R (antimicrobial activity)>2, the coating shows antibacterial activity. As it is shown in Table 1, antimicrobial activity (R) >2 at 24 h was confirmed for the coated samples.

**Table 1. In vitro antibacterial activity of coated samples against Staphylococcus epidermidis at time t=0 hours and t=24hours**

| | **t = 0h** | **t = 24h** | **R (log)** |
|---|---|---|---|
| **Uncoated samples (control)** | 2.7 * 10⁵ | 1.58 * 10⁵ | |
| | 3.13 * 10⁵ | 1.67 * 10⁵ | |
| | 2.65 * 10⁵ | 1.26 * 10⁵ | |
| **Coated samples** | 1.2 * 10⁵ | 5 * 10 | **3.56** |
| | 1.75 * 10⁵ | < 50 | |
| | 2.79 * 10⁵ | 7.5 * 10 | |

The biocompatibility of the coated stainless steel samples was assessed following ISO10993 standard (Biological evaluation of medical devices). Results (herein not shown) demonstrate that the coated samples are biocompatible in terms of cytotoxicity, irritability and sensitization.

### 1.3.2. In vivo assay: determination of the degree of osseointegration and antibacterial property of the coated implant L10 in rabbits

### In vivo experimental conditions

An *in vivo* test was performed in New Zealand rabbits with the uncoated implants and with the coated implants L10.

The *in vivo* test was performed following registered n. ANIM 10/13 test procedure, including the bio-statistical analyses of the collected data, approved by the Ethics Committee for Animal Research (200690050419), involving 13 animals with 6x13 uncoated samples; and 13 animals with 6x13 coated samples.

Briefly, the rabbits were first operated to insert the implants in their femur. Then, in order to obtain values about the antibacterial property of the coated implants L10, the rabbits were infected during 4 weeks with a daily inoculation of *S*. *Epidermidis* CECT 231 (10⁸ ufc/ml). No antibiotic was administered to the rabbits during the bacteria inoculation phase.

On the fifth week a femur was extracted from each rabbit and the coated implants L10 and the uncoated implants were extracted by a torque gauge in order to measure their degree of osseointegration (MARK-100 series TT03 digital torque gauge).

### Measurement of the degree of osseointegration

The results of Table 2 show the *in-vivo* degree of osseointegration of the coated implants L10 in comparison with the uncoated implants. The values shown in Table 2 correspond to the measured extraction torque that had to be applied to remove the implants from the femur. When a higher extraction torque is used, it means that the implant has a higher degree of osseointegration. According to the results, the statistical analysis (Student's t-test) of the differences in the extraction torques applied on the coated and uncoated implants (11.7±3.7 N.cm and 12.3±3.6 N.cm respectively) showed no statistically significant difference (p=0.6763 > 0.5). In view of the above, the coated implants L10 present a low-degree of osseointegration as the uncoated implants.

**Table 2: Results of the measured extraction torque for the coated implants L10 and uncoated implants (control)**

| **Implant** | **Extraction Torque (N.cm)** | | **Implant** | **Extraction Torque (N.cm)** |
|---|---|---|---|---|
| **Control** | 10.4 | | **Coated** | 10.4 |
| **Control** | 12.7 | | **Coated** | 12.7 |
| **Control** | 18 | | **Coated** | 10.3 |
| **Control** | 13.3 | | **Coated** | 10.7 |
| **Control** | 8.6 | | **Coated** | 7.1 |
| **Control** | 4.9 | | **Coated** | 7.6 |
| **Control** | 16.2 | | **Coated** | 13.9 |
| **Control** | 11.6 | | **Coated** | 8.6 |
| **Control** | 12.4 | | **Coated** | 13.3 |
| **Control** | 16.2 | | **Coated** | 14.3 |
| **Control** | 8.3 | | **Coated** | 17.8 |
| **Control** | 9.6 | | **Coated** | 18.2 |
| **Control** | 10.1 | | **Coated** | 15.2 |
| **Average** | **11.7** | | **Average** | **12.3** |
| **S.D.** | 3.7 | | **S.D.** | 3.6 |

### In vivo antibacterial activity results

The *in vivo* tests evaluated the performance of the coating of the present example deposited on CE marked 316L manufactured temporal external implants, in terms of:

### I. Presence of osteomyelitis by anatomopathology analysis

The hypothesis of the study was defined as H0: the probability of a severe or moderate infection is independent of the coated or uncoated external implant.

As it is shown in Table 3, the statistical analysis of the results (Fisher test) rejected the H0 hypothesis with p = 0.001, implying that differences among the coated external implants and uncoated external implants (control) in the anatomopathology analysis were statistically significant.

**Table 3. Results of anatomopatholgy analyses for coated external implants and uncoated external implants (control)**

| **Implant** | **Anatomopathology (Osteomyelitis)** | | **Implant** | **Anatomopathology (Osteomyelitis)** |
|---|---|---|---|---|
| **Control** | SEVERE | | **Coated** | MILD |
| **Control** | SEVERE | | **Coated** | MILD |
| **Control** | MODERATE | | **Coated** | MODERATE |
| **Control** | SEVERE | | **Coated** | MILD |
| **Control** | SEVERE | | **Coated** | MILD |
| **Control** | MILD | | **Coated** | MILD |
| **Control** | MILD | | **Coated** | MILD |
| **Control** | MILD | | **Coated** | MILD |
| **Control** | MILD | | **Coated** | MILD |
| **Control** | MILD | | **Coated** | MILD |
| **Control** | MILD | | **Coated** | MILD |
| **Control** | MILD | | **Coated** | MILD |
| **Control** | MODERATE | | **Coated** | MILD |

### II. Bacterial load by Scanning Electron Microscopy (SEM)

As in the previous point I., the hypothesis of the study was defined as H0: the probability of a severe or moderate infection is independent of the coated or uncoated external implant.

As it is shown in Table 4, the statistical analysis of the results (Fisher test) rejected the H0 hypothesis with p=0.00046, implying that differences among the coated and uncoated samples in the bacterial load SEM analysis were statistically strongly significant (p<0.001).

**Table 4. In vivo results of bacterial load SEM analyses on coated and uncoated (control) external implants**

| **Implant** | **SEM** | | **Implant** | **SEM** |
|---|---|---|---|---|
| **Control** | SEVERE | | **Coated** | MILD |
| **Control** | SEVERE | | **Coated** | MILD |
| **Control** | SEVERE | | **Coated** | MODERATE |
| **Control** | MODERATE | | **Coated** | MILD |
| **Control** | SEVERE | | **Coated** | MILD |
| **Control** | SEVERE | | **Coated** | MILD |
| **Control** | MILD | | **Coated** | MODERATE |
| **Control** | SEVERE | | **Coated** | MILD |
| **Control** | MODERATE | | **Coated** | MILD |
| **Control** | MODERATE | | **Coated** | MODERATE |
| **Control** | MODERATE | | **Coated** | MILD |
| **Control** | MODERATE | | **Coated** | MILD |
| **Control** | MODERATE | | **Coated** | MILD |

### Example 2 (not according to the invention)

The present example disclosed the preparation of coated samples (hereinafter referred as L6) and the evaluation of their *in vitro* antibacterial activity.

### 2.1. Preparation of TiₓAg_{y}N_{z}

TiₓAg_{y}N_{z} was prepared as in Example 1.

### 2.2. Coating a flat sample with TiₓAg_{y}N_{z}

Samples made of AISI 316L stainless steel with a flat geometry, i.e. 50 mm x 50 mm x 1 mm in size were used. Prior to the coating with the TiₓAg_{y}N_{z} obtained in point 2.1., flat samples were ultrasonically cleaned in isopropyl alcohol and finally dried.

Then, the TiₓAg_{y}N_{z} was deposited on the flat samples by arc PVD coating method. The distance between the flat samples and the TiₓAg_{y}N_{z} was 100 mm. The process was carried out at a pressure is 0.8 Pa. The deposition time was 4 minutes. The arc current applied was 90 A and the bias voltage was 80 V.

The deposition of the TiₓAg_{y}N_{z} by arc ion plating method on the samples provided a coating with a thickness of 0.9 µm measured according with the standard ISO 26423:2016-11.

Then, the coated samples were subjected to removal, at the surface, of metal droplets formed during the arc ion method. The method used to remove metal droplets is an abrasive drag grinding method that was performed during 6 minutes.

The X-ray photoelectron spectroscopy (XPS) analysis of the surface showed an Ag 3d5/2 peak at 368eV, corresponding to Ag0 metallic silver. Nitrogen 1s was present around 397-396 eV, which corresponds to Ti-N. As for Ti, there was present a peak around 457 eV associated with titanium oxide, TiO₂, and with Ti-N. The outer surface TiAgN chemical composition, as per XPS, presented 2.29 % at. of silver, 27.91 % at. of titanium, 48.71 % at. of oxygen, and 21.09 % at. of nitrogen. These data correspond to: Ag / (Ag + Ti) = 7.6 % at.

### 2.3. In vitro assay: measurement of the antibacterial property and the Ag lixiviation grade of the coated samples L6

The coated samples L6 were tested *in vitro* in order to prove their antibacterial properties and the non-lixiviation of the Ag from the coating.

First, the antibacterial activity was assessed *in vitro,* following the ISO 22196: 2011 (Measurement of antibacterial activity on plastics and other non-porous surfaces) against *Staphylococcus epidermidis, Escherichia Coli* and *Methicillin-resistant Staphylococcus aureus (MRSA).*

JIS Z 2801: 2000 (Antimicrobial products -Test for antimicrobial activity and efficacy) state that if R (antimicrobial activity) >2, the coating shows antibacterial activity. As it is shown in Table 5, antimicrobial activity (R) >2 was confirmed for the coated samples L6.

**Table 5. In vitro antibacterial activity of the coated samples (L6) against Staphylococcus epidermidis, Escherichia Coli and Methicillin-resistant Staphylococcus aureus (MRSA).**

| **Bacteria** | ***R (log)*** |
|---|---|
| *Staphylococcus epidermidis* | **4.9** |
| *Escherichia Coli* | **3.47** |
| *MRSA* | **4.8** |

Secondly, the quantity of Ag lixiviated from two equal coated samples L6 (referred as L6-1 and L6-2) was measured by ICP-MS analysis in bacterial culture media extracts (breeding media 1:500; 37°C; 24h). The results of Table 6 showed no Ag release, i.e. < 5 µg/L from the coated samples L6-1 and L6-2.

**Table 6. Ag release from coated samples L6-1 and L6-2 in bacterial culture media.**

| **Reference of the coated samples** | **Detected quantity of Ag (µg/L)** |
|---|---|
| L6-1 | < 5µg |
| L6-2 | < 5µg |

## Claims

1. A coating for a non-permanent transcutaneous implant comprising a TiₓAg_{y}N_{z} **characterized in that**:
a) (y/ (x+y)) is comprised between 0.0025 and 0.20;
b) the sum of x+y+z is equal to 1;
c) z is comprised between 0 and 0.55;
wherein x, y and z denote respectively the atomic ratios of Ti, Ag and N; and wherein the coating has a thickness comprised between 0.1 µm and 0.45 µm; and wherein the coating has a roughness of Ra comprised between 0.01 and 0.35 µm.

2. The coating according to claim 1, wherein z is comprised between 0.03 and 0.55, preferably between 0.2 and 0.55.

3. The coating according to any one of claims 1 or 2, wherein (y/(x+y)) is comprised between 0.0025 and 0.15, preferably between 0.0025 and 0.10.

4. The coating according to any one of claims 1 to 3, wherein the thickness is comprised between 0.15 and 0.45 µm, preferably 0.2 and 0.40 µm, more preferably 0.25 and 0.35 µm, for example 0.3 µm.

5. The coating according to any one of claims 1 to 4, wherein the roughness of Ra is comprised between 0.01 and 0.35 µm, preferably 0.01 and 0.30 µm, more preferably 0.01 and 0.28 µm, for example 0.01 and 0.25 µm.

6. The coating according to any one of claims 1 to 5 comprising TiₓAg_{y}N_{z} **characterized in that**:
a) (y/ (x+y)) is comprised between 0.02 and 0.045;
b) the sum of x+y+z is equal to 1;
c) z is comprised between 0 and 0.55;
wherein x, y and z denote respectively the atomic ratios of Ti, Ag and N; and wherein the coating has a thickness comprised between 0.25 µm and 0.35 µm; and wherein the coating has a roughness of Ra comprised between 0.04 and 0.25 µm.

7. The coating according to any one of claims 1 to 5 comprising TiₓAg_{y}N_{z} **characterized in that**:
a) (y/ (x+y)) is comprised between 0.016 and 0.077;
b) the sum of x+y+z is equal to 1;
c) z is comprised between 0 and 0.55;
wherein x, y and z denote respectively the atomic ratios of Ti, Ag and N; and wherein the coating has a thickness comprised between 0.1 µm and 0.2 µm; and wherein the coating has a roughness of Ra comprised between 0.037 and 0.094 µm.

8. A process for producing a coating for a non-permanent transcutaneous implant comprising the steps of:
a) evaporating an Ag alloyed Ti target in the presence of a gas selected from nitrogen, an inert gas, and mixtures thereof, obtaining TiₓAg_{y}N_{z} as defined in claims 1 to 7;
b) coating a transcutaneous implant until a thickness comprised between 0.1 µm and 0.45 µm with the TiₓAg_{y}N_{z} obtained in step a) by arc ion plating technology to obtain a coated implant;
c) removing surface metal droplets formed in step b) from the coated implant until a roughness of Ra comprised between 0.01 and 0.35 µm is achieved.

9. The process according to claim 8, wherein the transcutaneous implant of step b) is made of a material selected from the group consisting of stainless steel alloys, titanium alloys, biocompatible CoCr alloys, Polyether Ether Ketones and mixtures thereof.

10. The process according to any one of claims 8 to 9, wherein the arc ion plating method of step b) is carried out at a pressure of 0.7 to 5 Pa.

11. The process according to any one of claims 8 to 10, wherein step c) is carried out by abrasive drag grinding, abrasive filament brushing, magnetic particle abrasion finishing, abrasive dry or wet blasting.

12. A coated non-permanent transcutaneous implant comprising a transcutaneous implant and a coating as defined in claims 1 to 7 or obtained by the process of any of claims 8 to 11.

13. A coated non-permanent transcutaneous implant as defined in claim 12, for use in therapy.

14. A coated non-permanent transcutaneous implant as defined in claim 12, for use in the treatment of bone fractures and or in the prevention or treatment of osteomyelitis or bacterial infection.

15. The coated non-permanent transcutaneous implant according to claim 14, wherein the bacterial infection or osteomyelitis is caused by bacteria selected from the group consisting of *Staphylococcus epidermidis, Escherichia coli or Methicillin-resistant Staphylococcus aureus (MRSA).*

## Patentansprüche

1. Eine Beschichtung für ein nicht-permanentes transkutanes Implantat, umfassend ein TiₓAg_{y}N_{z}, **dadurch gekennzeichnet dass**:
a) (y/(x+y)) beträgt zwischen 0,0025 und 0,20;
b) die Summe aus x+y+z ist gleich 1;
c) z beträgt zwischen 0 und 0,55;
wobei x, y bzw. z die atomaren Verhältnisse zwischen Ti, Ag und N bezeichnen; und
wobei die Beschichtung eine Dicke zwischen 0,1 µm und 0,45 µm besitzt; und
wobei die Beschichtung eine Rauigkeit von Ra zwischen 0,01 und 0,35 µm aufweist.

2. Die Beschichtung gemäß Anspruch 1, wobei z zwischen 0,03 und 0,55 beträgt, vorzugsweise zwischen 0,2 und 0,55.

3. Die Beschichtung gemäß einem der Ansprüche 1 oder 2, wobei (y/(x+y)) zwischen 0,0025 und 0,15, vorzugsweise zwischen 0,0025 und 0,10 beträgt.

4. Die Beschichtung gemäß einem der Ansprüche 1 - 3, wobei die Dicke zwischen 0,15 und 0,45 µm beträgt, vorzugsweise 0,2 und 0,40 µm, besonders bevorzugt 0,25 und 0,35 µm, beispielsweise 0,3 µm.

5. Die Beschichtung gemäß einem der Ansprüche 1 - 4, wobei die Rauigkeit von Ra zwischen 0,01 und 0,35 µm beträgt, vorzugsweise 0,01 und 0,30 µm, besonders bevorzugt 0,01 und 0,28 µm, beispielsweise 0,01 und 0,25 µm.

6. Die Beschichtung gemäß einem der Ansprüche 1 - 5, umfassend TiₓAg_{y}N_{z} **dadurch gekennzeichnet dass**:
a) (y/(x+y)) beträgt zwischen 0,02 und 0,045;
b) die Summe aus x+y+z ist gleich 1;
c) z beträgt zwischen 0 und 0,55;
wobei x, y bzw. z die atomaren Verhältnisse zwischen Ti, Ag und N bezeichnen; und
wobei die Beschichtung eine Dicke zwischen 0,25 µm und 0,35 µm besitzt; und
wobei die Beschichtung eine Rauigkeit von Ra zwischen 0,04 und 0,25 µm aufweist.

7. Die Beschichtung gemäß einem der Ansprüche 1 - 5, umfassend TiₓAg_{y}N_{z} **dadurch gekennzeichnet dass**:
a) (y/(x+y)) beträgt zwischen 0,016 und 0,077;
b) die Summe aus x+y+z ist gleich 1;
c) z beträgt zwischen 0 und 0,55;
wobei x, y bzw. z die atomaren Verhältnisse zwischen Ti, Ag und N bezeichnen; und
wobei die Beschichtung eine Dicke zwischen 0,1 µm und 0,2 µm besitzt; und
wobei die Beschichtung eine Rauigkeit von Ra zwischen 0,037 und 0,094 µm aufweist.

8. Ein Verfahren zur Herstellung einer Beschichtung für ein nicht-permanentes transkutanes Implantat, umfassend die folgenden Schritte:
a) das Verdampfen eines Silber-legierten Titan-Anteils in Gegenwart eines Gases, ausgewählt aus Stickstoff, einem inerten Gas, und einer Mischung derselben, dadurch erhaltend TiₓAg_{y}N_{z} wie definiert in den Ansprüchen 1 - 7;
b) Beschichten eines transkutanen Implantats bis zu einer Dicke von zwischen 0,1 µm und 0,45 µm mit dem TiₓAg_{y}N_{z}, erhalten in Schritt a), durch Lichtbogen-Ionenplattierungs-Technologie, um ein beschichtetes Implantat zu erhalten;
c) Entfernen der Oberflächen-Metall-Tröpfchen, die sich im Schritt b) gebildet haben, von dem beschichteten Implantat, bis eine Rauigkeit von Ra zwischen 0,01 und 0,35 µm erreicht ist.

9. Das Verfahren gemäß Anspruch 8, wobei das transkutane Implantat von Schritt b) aus einem Material besteht, welches ausgewählt ist aus der Gruppe bestehend aus Edelstahl-Legierungen, Titan-Legierungen, biokompatiblen CoCr-Legierungen, Polyether Ether Ketonen und einer Mischung derselben.

10. Das Verfahren gemäß einem der Ansprüche 8 - 9, wobei das Lichtbogen-Ionenplattierungsverfahren von Schritt b) bei einem Druck von 0,7 bis 5 Pa ausgeführt wird.

11. Das Verfahren gemäß einem der Ansprüche 8 - 10, wobei Schritt c) durch abrasives Schlepp-Schleifen, abrasives Filament-Bürsten, magnetische Partikel-Abrasionsbehandlung, oder abrasive trockene oder nasse Abstrahlung ausgeführt wird.

12. Ein beschichtetes nicht-permanentes transkutanes Implantat, umfassend ein transkutanes Implantat und eine Beschichtung wie definiert in den Ansprüchen 1 - 7 oder erhalten durch das Verfahren gemäß einem der Ansprüche 8 - 11.

13. Ein beschichtetes nicht-permanentes transkutanes Implantat wie definiert in Anspruch 12 zur Verwendung bei einer therapeutischen Behandlung.

14. Ein beschichtetes nicht-permanentes transkutanes Implantat wie definiert in Anspruch 12 zur Verwendung bei der Behandlung von Knochenbrüchen und/oder bei der Prävention oder Behandlung von Osteomyelitis oder bakterieller Infektion.

15. Das beschichtete nicht-permanente transkutane Implantat gemäß Anspruch 14, wobei die bakterielle Infektion oder Osteomyelitis durch Bakterien, ausgewählt aus der Gruppe bestehend aus *Staphylococcus epidermidis, Escherichia coli* oder Methicillinresistenten *Staphylococcus aureus (MRSA),* verursacht wird.

## Revendications

1. Revêtement pour un implant transcutané non permanent comprenant un TiₓAg_{y}N_{z}, **caractérisé en ce que** :
a) (y/(x+y)) est compris entre 0,0025 et 0,20 ;
b) la somme x+y+z est égale à 1 ;
c) z est compris entre 0 et 0,55 ;
dans lequel x, y et z désignent respectivement les rapports atomiques de Ti, Ag et N ; et lequel revêtement a une épaisseur comprise entre 0,1 µm et 0,45 µm ; et lequel revêtement a une rugosité de Ra comprise entre 0,01 et 0,35 µm.

2. Revêtement selon la revendication 1, dans lequel z est compris entre 0,03 et 0,55, de préférence entre 0,2 et 0,55.

3. Revêtement selon l'une quelconque des revendications 1 ou 2, dans lequel (y/(x+y)) est compris entre 0,0025 et 0,15, de préférence entre 0,0025 et 0,10.

4. Revêtement selon l'une quelconque des revendications 1 à 3, dans lequel l'épaisseur est comprise entre 0,15 et 0,45 µm, de préférence entre 0,2 et 0,40 µm, plus préférentiellement entre 0,25 et 0,35 µm, par exemple 0,3 µm.

5. Revêtement selon l'une quelconque des revendications 1 à 4, dans lequel la rugosité de Ra est comprise entre 0,01 et 0,35 µm, de préférence entre 0,01 et 0,30 µm, plus préférentiellement entre 0,01 et 0,28 µm, par exemple entre 0,01 et 0,25 µm.

6. Revêtement selon l'une quelconque des revendications 1 à 5, comprenant TiₓAg_{y}N_{z}, **caractérisé en ce que** :
a) (y/(x+y)) est compris entre 0,02 et 0,045 ;
b) la somme x+y+z est égale à 1 ;
c) z est compris entre 0 et 0,55 ;
dans lequel x, y et z désignent respectivement les rapports atomiques de Ti, Ag et N ; et lequel revêtement a une épaisseur comprise entre 0,25 µm et 0,35 µm ; et lequel revêtement a une rugosité de Ra comprise entre 0,04 et 0,25 µm.

7. Revêtement selon l'une quelconque des revendications 1 à 5, comprenant TiₓAg_{y}N_{z}, **caractérisé en ce que** :
a) (y/(x+y)) est compris entre 0,016 et 0,077 ;
b) la somme x+y+z est égale à 1 ;
c) z est compris entre 0 et 0,55 ;
dans lequel x, y et z désignent respectivement les rapports atomiques de Ti, Ag et N ; et lequel revêtement a une épaisseur comprise entre 0,1 µm et 0,2 µm ; et lequel revêtement a une rugosité de Ra comprise entre 0,037 et 0,094 µm.

8. Procédé pour produire un revêtement pour un implant transcutané non permanent, comprenant les étapes de :
a) évaporation d'une cible en Ti allié d'Ag en présence d'un gaz choisi parmi l'azote, un gaz inerte, et leurs mélanges, pour obtenir TiₓAg_{y}N_{z} tel que défini dans les revendications 1 à 7 ;
b) revêtement d'un implant transcutané jusqu'à une épaisseur comprise entre 0,1 µm et 0,45 µm avec le TiₓAg_{y}N_{z} obtenu dans l'étape a) par une technologie de placage ionique à arc pour obtenir un implant revêtu ;
c) retrait des gouttelettes métalliques de surface formées dans l'étape b) sur l'implant revêtu jusqu'à ce qu'une rugosité de Ra comprise entre 0,01 et 0,35 µm soit atteinte.

9. Procédé selon la revendication 8, dans lequel l'implant transcutané de l'étape b) est fait en un matériau choisi dans le groupe constitué par les alliages d'acier inoxydable, les alliages de titane, les alliages de CoCr biocompatibles, les polyéther éther cétones et leurs mélanges.

10. Procédé selon l'une quelconque des revendications 8 à 9, dans lequel la méthode de placage ionique à arc de l'étape b) est mise en œuvre à une pression de 0,7 à 5 Pa.

11. Procédé selon l'une quelconque des revendications 8 à 10, dans lequel l'étape c) est mise en œuvre par meulage par traînée abrasive, brossage avec filament abrasif, finition par abrasion avec particules magnétiques, sablage abrasif à sec ou humide.

12. Implant transcutané non permanent revêtu comprenant un implant transcutané et un revêtement tel que défini dans les revendications 1 à 7 ou obtenu par le procédé de l'une quelconque des revendications 8 à 11.

13. Implant transcutané non permanent revêtu selon la revendication 12, pour une utilisation en thérapie.

14. Implant transcutané non permanent revêtu selon la revendication 12, pour une utilisation dans le traitement de fractures osseuses et ou dans la prévention ou le traitement d'une ostéomyélite ou d'une infection bactérienne.

15. Implant transcutané non permanent revêtu selon la revendication 14, dans lequel l'infection bactérienne ou l'ostéomyélite est provoquée par une bactérie choisie dans le groupe constitué par *Staphylococcus epidermidis, Escherichia coli* ou *Staphylococcus aureus* résistant à la méthicilline (SARM).
